Europäisches Patentamt

(19) European Patent Office

Office européen des brevets

(11) Numéro de publication: **0 035 937**
**B1**

(12) **FASCICULE DE BREVET EUROPEEN**

(45) Date de publication du fascicule du brevet:
07.09.83

(21) Numéro de dépôt: **81400332.3**

(22) Date de dépôt: **04.03.81**

(51) Int. Cl.³: **A 61 K 35/72, A 61 K 31/715**

(54) Utilisation de Hansenula ou d'extraits de Hansenula, comme médicament.

(30) Priorité: **10.03.80 FR 8005302**

(43) Date de publication de la demande:
**16.09.81 Bulletin 81/37**

(45) Mention de la délivrance du brevet:
**07.09.83 Bulletin 83/36**

(84) Etats contractants désignés:
**CH DE GB IT LI**

(56) Documents cités:
**FR-A-2 059 510**
**FR-A-2 290 218**
**FR-A-2 374 042**
**CHEMICAL ABSTRACTS, vol. 89, no. 19, 6 novembre 1978, réf. no. 161420q, page 411 COLUMBUS OHIO (US) HAMURO JUNJI et al.: »Glucanmediated augmentation of alloreactive murine cytotoxic T-lymphocytes in vivo«**

Le dossier contient des informations techniques présentées postérieurement au dépôt de la demande et ne figurant pas dans le présent fascicule.

(73) Titulaire: **UNIVABLOT, 21 bis rue Jonquoy, F-75014 Paris (FR)**

(72) Inventeur: **Massot, Jacqueline Olga, née Claude, 4 rue Prudhon, Pontault-Combault Seine-et-Marne (FR)**
Inventeur: **Astoin, Jacques Noel, 5 rue Basse des Carmes, Paris (FR)**

(74) Mandataire: **Casanova, André et al, CABINET ARMENGAUD JEUNE CASANOVA, AKERMAN, LEPEUDRY 23 boulevard de Strasbourg, F-75010 Paris (FR)**

## Utilisation de Hansenula ou d'extraits de Hansenula, comme médicament

La présente invention a pour objet l'utilisation de Hansenula notamment Hansenula anomala, ou d'extraits de Hansenula en tant que médicament.

Les Hansenula sont des levures appartenant à la famille de Saccharomycetaceae et à la sous-famille des Saccharomycoideae qui comprend de nombreux genres. Ces genres sont définis par exemple dans »The Yeasts« (LODDER, North Holland Publiching Company, 1970, pages 405 à 554). L'espèce Hansenula anomala qui a été plus particulièrement étudiée par la Demanderesse, est connue et accessible dans plusieurs collections de micro-organismes, par exemple dans le CENTRAALBUREAU VOOR SCHIMMEL CULTURES (DELFT, Pays-Bas) sous le N° CBS 110.

A la connaissance de la Demanderesse, les Hansenula n'ont jamais été étudiées en vue d'un usage thérapeutique. Des levures appartenant à un genre différent, tel que Saccharomyces cerevisiae, ont trouvé des applications thérapeutiques, en particulier comme protecteurs ou régénérateurs de la flore bactérinne intestinale.

Dans le brevet FR-A-2 059 510 et l'article Chemical Abstracts vol. 89, n° 19, ref n° 1 614 209q, on décrit des polysaccharides renfermant des liaisons $\beta$-(1,3) et possèdant une activité pharmaceutique.

Par ailleurs, certains auteurs ont étudié des extraits de Saccharomyces cerevisiae ou leurs glucanes dans leur action sur le système réticulo-endothélial (RIGGI et Di Luzio, Am. J. Physiol. [1961] 200, 2, pages 297 — 300 ou dans leur action antitumorale (HAMURO et coll., C.A., [1978] 89, 40 704 b).

Or, la Demanderesse a trouvé que les Hansenula, notamment Hansenula anomala ainsi que des extraits, en particulier les glucanes insolubles possèdent des propriétes pharmacologiques exceptionnelles justifiant leur application en tant que médicaments des maladies infectieuses: immunostimulation non spécifique, potentialisation de l'action des antibiotiques et augmentation de l'activité des vaccins.

Les souches de Hansenula peuvent être entretenues et cultivées de manière connue sur des milieux d'entretien et de fermentation habituellement utilisés pour les levures. Les cellules obtenues peuvent être séchées à 50° sous vide et de préference lyophilisées.

Les extraits selon l'invention, qui sont principalement des glucanes insolubles, peuvent être isolés de la paroi des cellules de Hansenula, par exemple selon la méthode indiquée par Bell et Northcote (J. Chem. Soc. [1950], pages 1944 — 47), éventuellement modifiée, par exemple selon Peat et coll. (idem, 1958a, pages 3862 — 3868). Le principe consiste à éliminer de la masse cellulaire obtenue par fermentation, d'abord les protéines et les lipides et les mannanes par traitement alcalin, puis le glycogène et les glucanes solubles par traitement acide.

Les exemples suivants illustrent l'obtention de ces produits.

### Exemple 1

#### Procédé de fermentation de Hansenula anomala

##### a) Préparation des pieds de cuve

On prépare le pied de cuve à partir de la culture d'entretien de la souche de Hansenula anomala. Celle-ci est obtenue par incubation pendant 48 heures à 30° C de la levure sur pente gélosée contenant:

Milieu (1)

| | |
|---|---|
| eau de levure | 500 ml |
| peptone pancréatique | 2 g |
| glucose massé | 10 g |
| gélose | 18 g |
| eau ordinaire q.s.p. | 1 litre |

On décolle la culture de la pente gélosée par environ 10 ml de sérum physiologique stérile par tube. On verse la suspension ainsi obtenue de manière stérile dans un erlenmeyer de 6 litres contenant 1,5 litre de milieu de fermentation ayant la composition:

Milieu (2)

| | |
|---|---|
| glucose | 15 g |
| extrait de levure | 2,5 g |
| sulfate d'ammonium | 5 g |
| phosphate monopotassique | 5 g |
| sulfate de magnésium (7 $H_2O$) | 0,05 g |
| chlorure de calcium | 0,01 g |
| sulfate ferreux (7 $H_2O$) | 0,01 g |

| chlorure de potassium | 0,01 g |
| eau ordinaire q.s.p. | 1 litre |

L'incubation dure 24 heures à 27° ±1° sur agitateur rotatif à environ 115 t/minute.

## b) Fermentation

On ensemence le contenur de 2 erlenmeyer tel qu'obtenu ci-dessus (environ 3 litres) dans une cuve de fermentation stérile remplie de 200 litres de milieu (2) décrit ci-dessus. On effectue la fermentation pendant 20 heures sous aération de 20 m³/heure. Ensuite on centrifuge le contenu de la cuve afin de récolter la masse contenant les cellules de Hansenula.
On obtient 3,35 kg de masse cellulaire (humidité 78%).

## Exemple 2

### Obtention de cellules desséchées de Hansenula anomala

En séchant à 50°C sous vide 1 kg de la masse cellulaire obtenue dans l'exemple 1, on obtient 220 g de cellules prêtes à être utilisées.
Une variante, pour obtenir un produit plus facile à conserver et à utiliser, consiste à lyophiliser la masse cellulaire obtenue dans l'exemple 1; à partir de 1 kg de masse cellulaire on obtient ainsi 215 g de cellules utilisables.

## Exemple 3

### Obtention de glucanes de Hansenula anomala

On traite 1 kg de masse cellulaire fraîchment centrifugée obtenue selon l'exemple 1, par 3 litres d'une solution de soude à 6% pendant 1 heure 30 à 80°C; on recueille le résidu par centrifugation (4000 t/minute pendant 15 minutes) et on traite ensuite par 5 litres d'une solution de soude à 3% pendant 18 heures à température ambiante; on centrifuge à nouveau et on traite le produit insoluble de nouveau par une solution de soude à 3% pendant 1 heure 30 à 80°C. On centrifuge et on reprend le culot de centrifugation par 3 litres d'eau. On amène la suspension ainsi obtenue à pH 4,5 par le l'acide acétique (ou chlorhydrique) et on porte à 80°C pendant 2 heures sous agitation. On lave le résidu obtenu par centrifugation 3 fois par 1 litre d'eau bouillante et reprend par 2,5 litres d'une solution d'acétate de sodium 0,02 M puis procède à un autoclavage à 2 bars pendant 1 heure.
On lave la partie insoluble à l'eau, puis à l'éthanol, à l'acétone et on sèche sous vide à 50°C maximum. On obtient après broyage du produit obtenu 18,3 g d'une poudre crème.
L'alternative consiste à lyophiliser. A partir de la même quantité de cellules on obtient 15,3 g d'extrait.

Analyse:
   $C = 46,14\%$; $H = 7,72\%$; $O = 45,52\%$; $N = <0,4\%$; P: absent.

L'action de l'amylase d'origine pancréatique sur l'extrait ne libère que des traces de composés réducteurs, indiquant l'absende de glycogène.
L'extrait selon l'invention est remarquable par son insolubilité dans l'eau, les solutions alcalines ou acides, et les solvants organiques usuels, tels que l'éthanol, l'éther diéthylique, l'acétate d'éthyle, l'acétone, le chloroforme, etc.
On constate toutefois une très légère solubilité dans le diméthylsulfoxyde (DMSO).
Pouvoir rotatoire spécifique: $[\alpha]_D^{20} \simeq -65°$ (C = 0,16; DMSO).
Infrarouge: le spectre obtenu avec pastille de KBr présente une absorption caractéristique à 890 cm$^{-1}$.
Il ressort de ces données que les extraits obtenus de la paroi de Hansenula anomala sont principalement des polymères ramifiés de glucoses, liés par une majorité de liaisons $\beta$. L'action de l'hexo-$\beta$-(1—3)-D-glucanase sur l'extrait libère des composés réducteurs confirmant ainsi l'existence de liaisons $\beta$-(1—3). L'extrait selon l'invention est donc constitué essentiellement de $\beta$-(1—3)-D-glucanes qui seront appelés simplement »glucanes« dans le texte.
Les levures Hansenula, en particulier Hansenula anomala ainsi que les glucanes qui en sont extraits ont été soumis à des essais pharmacologiques.

## Toxicité

Aucune toxicité n'a été constatée chez la souris par voie orale, aussi bien pour Hansenula anomala que pour ses glucanes.

Par voie I.P., la $DL_{50}$ de ces glucanes est supérieure à 500 mg/kg.

## Mise en évidence de l'Immunostimulation par les cellules entières de Hansenula et par ses glucanes

### 1) par la consommation du complément

L'augmentation de la consommation du complément est le signe d'une stimulation immunitaire. Ce test a été effectué avec des cellules entières d'Hansenula anomala voie parentérale, les animaux utilisés sont des lapins. Ceux-ci sont répartis comme suit:

— 1 lot témoin de 2 lapins,
— 1 lapin recevant une injection parentérale de 5 mg/kg de Hansenula anomala,
— 1 lapin recevant une injection parentérale de 1 mg/kg.

Quotidiennement, la consommation du complément est dosée. Le dosage est traduit en finalité par la lecture de l'aire d'hémolyse, celle-ci étant le reflet de l'activité du complément des animaux.

Cet essai a donné les résultats suivants (en aires d'hémolyse).

| | Avant | $2^{ème}$ jour | $3^{ème}$ jour | $4^{ème}$ jour | $5^{ème}$ jour | $8^{ème}$ jour |
|---|---|---|---|---|---|---|
| Témoin N° 1 | 25,50 | 23,75 | 29,22 | 27,33 | 27,33 | |
| Témoin N° 2 | 26,41 | 22,89 | 24,62 | 22,04 | 23,75 | |
| Hansenula 5 mg | 29,22 | 36,32 | 50,27 | 39,60 | 33,18 | 38,18 |
| Hansenula 1 mg | 34,21 | 45,36 | 55,42 | 52,81 | 47,78 | 24,62 |

Comme on peut le constater, 48 heures après l'injection il y a augmentation très significative de l'activité du complément ( +70% pour 5 mg/kg; +61% pour 1 mg/kg).

### 2) par la protection contre une infection expérimentale mortelle traitement préventif; voie I.P.

Les animaux utilisés sont des souris SPF IFFA CREDO, femelles, de 18 — 20 grammes, nourries avant expérience durant 8 jours avec un régime dépourvu de levures mais contenant la dose de vitamines nécessaires.

Ces souris sont réparties en 2 lots:

— 1 lot témoin d'infection,
— 1 lot infecté et traité soit par Hansenula soit par ses glucanes.

Les expériences ont été effectuées sur les souris infectées par voie I.V. par environ $1 \times 10^6$ Staphylococcus aureus pathogènes (souche Institut Pasteur 54.146) ou par environ $1 \times 10^4$ Klebsiella pneumoniae.

Le traitement a été effectué par voir I.P.

— pour Hansenula anomala à la dose de 50 mg/kg,
— pour les glucanes de Hansenula à 10 mg/kg.

Le schéma opératoire est le suivant:

|  | Lot témoin | Lot traité |
|---|---|---|
| Jour — 7 | Injection I.P. de 0,2 ml de sérum physiologique par souris | Injection I.P. de 0,2 ml de suspension de Hansenula ou de glucanes |
| Jour — 4 | 2ème injection idem | 2ème injection idem |
| Jour — 0 | Infection bactérienne | |

Le nombre de souris mortes dans chaque lot est noté chaque jour.
Les tableaux suivants indiquent le nombre d'animaux survivants.

A) — Avec levures entières de Hansenula anomala

|  | Lot témoin | Lot traité |
|---|---|---|
| Infection par Klebsiella pneumoniae | 1/10 | 7/9 |
| % de survie | 10% | 77% |
| Infection par Staphylococcus aureus | 0/10 | 7/10 |
| % de survie | 0% | 70% |

B) — Avec les glucanes d'Hansenula

| Infection par Staphylococcus | Lot témoin | Lot traité |
|---|---|---|
| 1$^{er}$ essai | 0/10 | 8/10 |
| 2$^{ème}$ essai | 0/10 | 6/10 |
| 3$^{ème}$ essai | 2/10 | 7/10 |
| 4$^{ème}$ essai | 2/10 | 5/10 |
| 5$^{ème}$ essai | 2/10 | 7/10 |
| % de survie | 12% | 66% |

Comme on peut le constater, le nombre de souris mortes est inférieur dans les lots traités au nombre de souris mortes dans les lots témoins, que ce soit avec les cellules entières de Hansenula anomala ou avec ses glucanes.

Etant donné la virulence du germe injecté, la protection conférée par l'injection I.P. de Hansenula ou de ses glucanes est très importante. Cette protection est attribuable à une immunostimulation non spécifique.

### Mise en évidence de l'IMMUNOSTIMULATION par Hansenula anomala et par ses glucanes après injection préalable d'antigène

Les animaux utilisés sont des souris SPF IFFA CREDO, femelles, de 18—20 grammes, nourries avant l'expérimentation durant une semaine avec un régime ne comportant aucune levure mais contenant la dose nécessaire de vitamines.
Ces souris sont réparties en 5 lots:

— lot 1: témoin d'infection,
— lot 2: témoin antigène (»vaccin«),

- lot 3: recevant l'antigène + l'infection,
- lot 4: comme lot 3, avec en supplément un traitement par Hansenula anomala,
- lot 5: comme lot 3, avec en supplément un traitement par glucanes de Hansenula.

L'antigène est ici constitué d'une culture de staphylococcus aureus (Institut Pasteur 54.146) à dose non mortelle.

La culture de 16 heures en bouillon nutritif est diluée au moment de la vaccination à $1 \times 10^{-4}$, représentant environ $1 \times 10^4$ germes/ml.

Les souris des lots 1, 3 à 5 sont infectées par la souche très pathogène de staphylococcus aureus (Institut Pasteur 54.146) réactivée. Une culture en bouillon nutritif, incubée durant 16 heures à 37° est injectée par la voie I.V. pour infecter les souris après dilution au 1/100e dans du sérum physiologique (soit $1 \times 10^6$ germes/ml).

Le schéma opératoire est le suivant:

|  | lot 1 témoin d'infection | lot 2 témoin vaccin | lot 3 vaccin + infection | lot 4 idem 3 + Hansenula | lot 5 idem 3 + glucanes |
|---|---|---|---|---|---|
| Jour −9 | 0,2 ml I.V. de sérum physiologique | 0,2 ml I.V. de $10^4$ germes/ml (vaccin) | | | |
| Jour −6 à Jour −1 | 0,2 ml I.P. de sérum physiologique par jour | | | 0,2 ml I.P. 50 mg/kg Hansenula | 0,2 ml I.P. 10 mg/kg glucanes |
| Jour 0 | Infection ($10^6$ germes) 0,2 ml I.V. | sérum physio-logique 0,2 ml I.V. | Infection par voie I.V. 0,2 ml ($10^6$ germes) idem lot 1 | | |

Le nombre de souris mortes dans chaque lot est noté chaque jour. Les survies pour chaque lot ont été les suivantes:

- témoins d'inocuité du vaccin — 9/9 soit 100% de survie
- souris témoins d'infection — 2/10 soit 20% de survie
- souris vaccinées et infectées — 4/9 soit 44% de survie
- souris traitées en plus par Hansenula — 9/10 soit 90% de survie
- souris traitées en plus par glucanes — 9/10 soit 90% de survie

Ces résultats montrent que 80% des animaux infectés par $10^6$ Staphylococcus aureus sont morts. Les animaux, s'ils ont été vaccinés 1 fois, 9 jours avant cette infection sont en partie protégés puisque la mortalité est alors de 44%. Comme on peut le constater ici, l'addition à la vaccination d'un traitement par Hansenula ou par ses glucanes par la voie I.P. abaisse très fortement la mortalité à 10%.

Etant donné l'extrême virulence du germe injecté, la potentialisation conférée par les glucanes à une prévaccination spécifique a été importante. Cette potentialisation est là encore attribuable à une immunostimulation.

## Mise en évidence de la potentialisation de l'action des antibiotiques

Dans cet essai on a adjoint à un traitement antibiotique (chlorhydrate de tétracycline 4 jours à 10 mg/kg), un traitement préventif en 2 injections par des glucanes à la dose de 10 mg/kg.

Les animaux utilisés sont de la même souche qu'aux essais précédents et ont le même régime alimentaire.

Les souris sont réparties en 4 lots de souris:

- lot 1: témoin d'infection,
- lot 2: traité par glucanes de Hansenula,
- lot 3: traité par 10 mg/kg par jour de tétracycline,
- lot 4: recevant
    - glucanes de Hansenula,
    - 10 mg/kg par jour de tétracycline.

0 035 937

L'essai a été effectué sur des souris infectées par voie I.V. par environ $1 \times 10^6$ Staphylococcus aureus pathogènes (Institut Pasteur 54.146) réactivés.

Une culture en bouillon nutritif incubée durant 16 heures à 37°C est utilisée après dilution au 1/100e pour infecter les souris.

Le schéma opératoire est le suivant:

|  | témoins d'infection | glucanes | tétracycline | tétracycline + glucanes |
|---|---|---|---|---|
| Jour −7 à Jour −4 | I.P.: 0,2 ml de sérum physiologique | I.P.: 0,2 ml de glucanes 10 mg/kg | idem témoins | I.P.: 0,2 ml de glucanes 10 mg/kg |
| Jour 0 | INFECTION Staphylococcus voie I.V. | | | |
| Jour +1 à Jour +4 | Pers os: 0,2 ml de sérum physiologique | Per os: idem témoins | Per os: 0,2 ml de tétra-cycline | Per os: 0,2 ml de tétra-cycline |

Le nombre de souris mortes est relevé chaque jour. Le tableau suivant indique les survies observées pour chaque lot.

(voir page suivante)

| Témoins | glucanes seuls | tétracycline | glucanes + tétracycline |
|---|---|---|---|
| 1/10 | 3/10 | 2/10 | 4/10 |

Comme précédemment, il y a eu protection par les glucanes de Hansenula seuls. La tétracycline, à cette dose, a faiblement protégé les animaux (2/10). L'adjonction de glucanes à cette même dose de tétracycline a potentialisé l'action antibiotique de la tétracycline (4/10).

Les essais relatés ci-dessus ont mis en évidence que l'administration de Hansenula anomala ou de ses glucanes, d'une part, diminue par immunostimulation non spécifique la mortalité d'animaux infectés par un germe pathogène et, d'autre part, potentialise l'action d'antibiotiques et celle de vaccins. Cette levure est donc susceptible d'être utilisée pour renforcer la résistance de l'organisme contre les agents infectieux et pour améliorer les défenses immunitaires.

Les levures Hansenula anomala et les glucanes qui en sont extraits étant dépourvus de toxicité peuvent donc être utilisés dans la prévention et/ou le traitement des maladies infectieuses aiguës ou chroniques, en particulier des maladies de la sphère ORL et/ou pulmonaire, mais aussi de toute autre maladie d'origine virale ou bactérienne.

Hansenula anomala et ses glucanes peuvent être utilisés comme médicaments seuls ou en association avec un ou plusieurs antigènes ou encore en association avec un ou plusieurs antibiotiques.

Les cellules entières de Hansenula peuvent être administrées par voie orale à des doses quotidiennes de 100 à 2000 mg (produit sec) par jour, de préférence dans des gélules contenant les cellules lyophilisées et éventuellement des excipients usuels, ou bien en ampoules buvables, les cellules étant en suspension dans leur milieu de culture.

Exemple de composition d'une gélule:

| — | Cellules lyophilisées de Hansenula anomala | 50 mg |
| — | Lactose | 7 mg |
| — | Stéarate de magnésium | 2 mg |
| — | Sucre glace q.s.p. une gélule de | 150 mg |

Les glucanes de Hansenula peuvent aussi être administrés par voie orale, à des doses quotidiennes de 20 à 500 mg, ou seront utilisés également sous forme de suspensions injectables, à raison de 5 à 100 mg par jour. On peut aussi envisager l'usage de ces glucanes sous forme de crèmes dermiques ou d'aérosols.

7

Exemple de composition de suspension injectable:

|  |  |  |
|---|---|---|
| — | Glucanes de Hansenula anomala*) micronisés | 0,010 g |
| — | Polysorbate 80 | 0,025 g |
| — | Polyvinylpyrrolidone | 0,025 g |
| — | Phosphate monosodique | 0,025 g |
| — | Chlorure de sodium q.s.p. isotonie | |
| | methiolate de sodium | 0,0002 g |
| — | Eau distillée apyrogène q.s.p. | |
| | 1 ampoule injectable de 5 ml | |

*) (particules) < 50 μm dont 80% < 10 μm).

Exemple d'aérosol:

|  |  |  |
|---|---|---|
| — | Glucanes micronisés*) | 0,050 g |
| — | Polysorbate 80 | 0,2 g |
| — | Glycérides oléiques polyoxy-éthylènes | 1 g |
| — | Phosphate monosodique | 0,1 g |
| — | Chlorure de sodium q.s.p. isotonie | |
| | merthiolate de sodium | 0,0008 g |
| — | Eau purifiée . . . q.s.p. | 20 ml |

## Revendications

1. Substance constituée de Hansenula ou d'extraits de Hansenula utilisables pour le traitement des maladies infectieuses, immunostimulation non spécifique, potentialisation de l'action des antibiotiques et augmentation de l'activité des vaccins.

2. Substance selon la revendication 1, caractérisée par le fait qu'elle est constituée de Hansenula anomala ou d'extraits de Hansenula anomala.

3. Substance selon la revendication 2, caractérisée par le fait qu'elle est constituée de cellules entières de Hansenula anomala.

4. Substance selon la revendication 2, caractérisé par le fait qu'elle est constituée d'extraits de paroi de Hansenula anomala obtenus par un procédé consistant à éliminer des cellules de Hansenula anomala, les protéines, les lipides et les mannanes par traitement alcalin, puis le glycogène et les glucanes solubles par traitement acide.

5. Substance à base d'extraits selon les revendications 2 ou 4, caractérisée par le fait que les extraits sont principalement constitués de glucanes insolubles.

6. Substance selon la revendication 5, caractérisée par le fait que les glucanes insolubles sont essentiellement des $\beta$-(1 − 3)-D-glucanes.

7. Composition pharmaceutique renfermant à titre de substance active de l'Hansenula ou des extraits de Hansenula tels que définis dans l'une quelconque des revendications 1 à 6, ainsi qu'un véhicule pharmaceutiquement acceptable.

8. Composition pharmaceutique selon la revendication 7, administrable par voie orale, caractérisée par le fait qu'elle contient des cellules entières de Hansenula ou d'extraits de Hansenula.

9. Composition pharmaceutique selon la revendication 7, administrable par voie parentérale, caractérisée par le fait qu'elle contient des glucanes insolubles de Hansenula.

10. Composition pharmaceutique selon la revendication 7, sous forme d'aérosol, caractérisée par le fait qu'elle contient des glucanes insolubles de Hansenula.

## Patentansprüche

1. Substanz zur Behandlung von Infektionskrankheiten, zur nichtspezifischen Immunstimulation, zur Verstärkung der Wirkung von Antibiotika und zur Erhöhung der Wirkung von Vaccinen, dadurch gekennzeichnet, daß sie aus Hansenula oder Hansenula-Extrakten besteht.

2. Substanz nach Anspruch 1, dadurch gekennzeichnet, daß sie aus Hansenula anomala oder Hansenula anomala-Extrakten besteht.

3. Substanz nach Anspruch 2, dadurch gekennzeichnet, daß sie aus ganzen Zellen von Hansenula anomala besteht.

4. Substanz nach Anspruch 2, dadurch gekennzeichnet, daß sie aus Zellwandextrakten von Hansenula anomala besteht, die nach einem Verfahren erhalten worden sind, welches darin besteht, daß man die Zellmassen von Hansenula anomala, die Proteine, die Lipide und die Mannane durch alkalische Behandlung und danach das Glykogen und die löslichen Glucane durch saure Behandlung eliminiert.

5. Substanz in Form eines Extraktes nach einem der Ansprüche 2 oder 4, dadurch gekennzeichnet, daß der Extrakt im wesentlichen aus unlöslichen Glucanen besteht.

6. Substanz nach Anspruch 5, dadurch gekennzeichnet, daß diese unlöslichen Glucane im wesentlichen $\beta$-(1 – 3)-D-Glucane sind.

7. Pharmazeutisches Präparat, dadurch gekennzeichnet, daß es als wirksame Substanz Hansenula oder Hansenula-Extrakte gemäß einem der Ansprüche 1 bis 6 sowie einen pharmazeutisch akzeptablen Träger enthält.

8. Pharmazeutisches Präparat nach Anspruch 7, welches auf oralem Wege verabreichbar ist, dadurch gekennzeichnet, daß es ganze Zellen von Hansenula oder Hansenula-Extrakte enthält.

9. Pharmazeutisches Präparat nach Anspruch 7, welches auf parenteralem Wege verabreichbar ist, dadurch gekennzeichnet, daß es unlösliche Glucane aus Hansenula enthält.

10. Pharmazeutisches Präparat nach Anspruch 7, dadurch gekennzeichnet, daß es unlösliche Glucane aus Hansenula enthält.


**Claims**

1. Substance consisting of Hansenula or extracts of Hansenula that can be used in the treatment of infectious diseases, non-specific immunostimulation, potentialisation of the action of antibiotics, and enhancing the effect of vaccines.

2. Substance according to Claim 1, characterised in that it consists of Hansenula anomala or extracts of Hansenula anomala.

3. Substance according to Claim 2, characterised in that it consists of whole cells of Hansenula anomala.

4. Substance according to Claim 2, characterised in that it consists of Hansenula anomala cell wall extracts obtained by a process consisting of removing from Hansenula anomala cells, proteins, lipids and mannans by alkaline treatment, and then glycogen and soluble glucanes by acidic treatment.

5. Substances based on extracts according to Claim 2 or 4, characterised in that the extracts mainly consist of insoluble glucanes.

6. Substance according to Claim 5, characterised in that the insoluble glucanes are essentially beta-(1 – 3)-D-glucanes.

7. Pharmaceutical composition containing as active principle Hansenula or extracts of Hansenula as defined in any one of Claims 1 to 6, as well as a pharmaceutically acceptable vehicle.

8. Pharmaceutical composition according to Claim 7 that can be administrered orally, characterised in that it contains whole cells of Hansenula or extracts of Hansenula.

9. Pharmaceutical composition according to Claim 7 that can be administered parenterally, characterised in that it contains insoluble glucanes of Hansenula.

10. Pharmaceutical composition according to Claim 7, in the form of an aerosol, characterised in that it contains insoluble glucanes of Hansenula.